# EUROPEAN PATENT SPECIFICATION

(11) **EP 0 446 225 B1**
(45) Date of publication and mention of the grant of the patent: **03.03.1993**
(21) Application number: 89912676.7
(22) Date of filing: 22.11.1989
(51) Int. Cl.: A61K 31/165, A61K 31/075, A61K 31/21

(54) **MEDICAMENT FOR TREATMENT OF PATHOLOGICAL VASCULAR PERMEABILITY AND PLASMA LOSS TO TISSUE**
MEDIKAMENT ZUR BEHANDLUNG VON PATHOLOGISCHER GEFÄSSPERMEABILITÄT UND VON PLASMAVERLUST INS GEWEBE
MEDICAMENT POUR TRAITER DES PATHOLOGIES TELLES QUE LA PERMEABILITE VASCULAIRE ET LA DEPERDITION PLASMATIQUE TISSULAIRE

(30) Priority: 22.11.1988 SE 8804214
(43) Date of publication of application: 18.09.1991
(73) Proprietor: CASSUTO, Jean, S-413 15 Göteborg (SE); HEDNER, Thomas, S-412 67 Göteborg (SE)
(72) Inventor: CASSUTO, Jean, S-413 15 Göteborg (SE); HEDNER, Thomas, S-412 67 Göteborg (SE)
(74) Representative: Mossmark, Anders
(86) International application number: SE8900680
(87) International publication number: WO9005542

(56) References cited:
- EP-A-01 543 44
- FR-M- 3 385
- GB-A- 2 177 918
- US-A- 3 337 406
- US-A- 4 181 725
- US-A- 4 466 973
- US-A- 4 618 490
- US-A-43 339 41
- Chemical Abstracts, volume 101, no. 15, 8 October 1984, (Columbus, Ohio, US), C. Matei-Vladescu et al: "The effects of gerovital H3 treatment on antigen-induced arthritis in rabbits", see page 32, abstract 122717p
- Chemical Abstracts, volume 86, no. 17, 25 April 1977, (Columbus, Ohio, US), A.I. Frolova et al: "Effect of novocaine blockade on cardiac rhythm and stomach and intestinal biopotential in strangulated ileus", see page 39, abstract 115211n
- Chemical Abstracts, volume 104, no. 17, 28 April 1986,(Columbus, Ohio, US), I. Neascu: "The influence of procaine, gerovital and aslavital on cellular cholesterol-loaded membranes", see page 7, abstract 141615g
- Acta Physiol Scand, vol. 127, 1986 C.G.A. Persson et al: "Leakage of macromolecules from guinea-pig tancheobronchial microcirculation. Effects of allergen, leukotrienes, tachykinins and anti-asthma drugs", see page 95-105
- Agents and Actions, vol. 16, no. 1/2, 1985 I. Erjefält et al: "Anti-asthma drugs and capsaicineinduced microvascular effects in lower airways", see p. 9-page 10
- Chemical Abstracts, volume 96, no. 23, 7 June 1982, (Columbus, Ohio, US), J.R. Fletcher et al: "The protective effect of lidocaine on arachidonate metabolism in septic schock", see page 45, abstract 193134k
- Proc. Natl. Acad. Sci., vol. 78, no. 6, June 1981 D.K. Miller et al: "Antihistaminics, local anaesthetics and other amines as antiviral agents", see page 3605-page 3609
- Chemical Abstracts, volume 78, no. 1, 8 January 1973, (Columbus, Ohio, US), A.I. Krotov et al: "Experimental study of the effectiveness of combinations of fenasal with cucurubine and novocaine in treatment of hymenolepidosis of white mice", see page 16, abstract 188c
- Agents Actions, vol. 13, no. 1, 1983 D.F. Woodward et al: "Studies on histamine-induced cutaneous flare in the guinea pig", see page 35-page 44
- Arzneimittelforschung, vol. 34, no. 7, 1984 P.Schieper: "Comparative studies of local anesthetic effects on artificial membranes and isolated cardiac tissues", see page 759-page 761
- Chemical Abstracts, volume 105, no. 9, 1 September 1986, (Columbus, Ohio, US), R.White et al: "Tocainide suppression of immune-complex-mediated dermal inflammation: comparasion with prostaglandin E1", see page 35, abstract 72272z, & Clin. Immunol. Immunopathol. 1986, 39(3), 452-63
- Chemical Abstracts, volume 108, no. 9, 29 February 1988, (Columbus, Ohio, US), E. Hardie et al: "Lidocaine treatment of dogs with Escherichia coli septicemia", see page 52, abstract 68647z,

## Description

The present invention relates to previously unreported properties of known local anaesthetic drugs, which properties involve an inhibition of increased permeability in the blood vessels, which contributes to preventing increased plasma loss to tissue in association with certain pathological conditions.

These newly discovered properties of local anaesthetic drugs therefore mean that the substances in question can be used in pathological conditions where local anaesthetics have hitherto not been used, but instead other pharmacological or surgical treatment forms have predominated.

### Background of the invention:

Loss of circulating protein components and cellular elements from plasma constitutes an initial and very important subcomponent in many pathological conditions where damage of various tissues occurs as a result of external action.

The type of pathological condition to which the patent application relates can occur as a result of non-specific action such as, for example, heat, fire, cold and radiation or as a result of microorganisms, for example viruses, bacteria, fungi and parasites.

These external agents or damaging processes can therefore, by means of similar initial mechanisms, initiate a sequence of biological processes which mean that irreversible tissue damage occurs. This tissue damage is caused by the body's own biological defence processes which, in the event of excessive activation, can have damaging consequences. Of course, the particular reaction or reactions initiated initially have purposeful components which aim to limit the damaging or pathological processes.

In association with a damage process of the abovementioned type, a direct loss of chemical substances from plasma occurs in the damaged area. This initial stage is followed by local accumulation of white blood cells which produce biological mediators of a chemical nature which can act on and limit the subsequent bacterial invasion. This gradually leads to the process of tissue damage being counteracted, and the subsequent healing process is promoted. However, the inflammatory and healing process is a subsequent phenomenon which succeeds the initial protein loss following the injury. However, these later inflammation and healing phenomena do not come within this patent application.

To sum up, the loss of plasma constituents can often lead to reactions which can be damaging to the individual, since uncontrolled, non-purposeful injury processes follow this initial process in those tissues or organs affected by the external injury.

The particular types of damage processes to which the present invention can relate occur in the case of burn injuries, cold injuries or chemical injuries of the skin or internal organs, in particular in the abdominal cavity or thoracic cage.

Disorders which can arise in the case of damage processes of this type are nowadays treated by medical/pharmacological means or by surgery. The pharmacological treatment comprises a number of different drug groups such as, for example, antibiotics and chemotherapeutic agents, NSAIDs (NonSteroid AntiInflammatory Drugs), steroids and cytostatic agents. Surgical therapy most often involves resection of tissue or parts of organs.

The drugs which are used nowadays in the treatment of these injury processes have more or less serious side effects, which often seriously limit their use over prolonged periods. These side effects can comprise, in the case of antibiotics or NSAIDS, serious allergic reactions and, in the case of steroids, for example gastric ulcers, brittle bones and increased sensitivity to infection.

### Aim of the invention:

The aim of the invention is to provide a medicament and a treatment programme which counteracts the injury process at a very early stage. This means that the tissue damage can be limited, which in turn means that recourse need not be made to the abovementioned medical/pharmacological or surgical treatment methods. By using local anaesthetics for these new indications, it is possible to limit the protein loss and, therefore, to promote the body's own healing processes without the abovementioned disadvantages and side effects of other drug therapy or surgical treatment.

### Characteristics of the invention:

This aim is achieved by the present invention, which is based on the use of a chemical local anaesthetic agent selected from the local anaesthetic compound groups: esters, for example BENZOCAINE, PROCAINE and TETRACAINE, amides, for example CINCHOCAINE, LIDOCAINE, PRILOCAINE, MEPIVACAINE, BUPIVACAINE and ETIDOCAINE, ethers, for example CHINISOCAINE and PRAMOCAINE, for the production of a medicament for prophylactic or therapeutic inhibition of increased vascular permeability in the blood vessels, which contributes to preventing increased plasma loss to tissue in the case of burn injuries, cold injuries or chemical injuries of the skin or internal organs, in particular in the abdominal cavity or thoracic cage. Because the injury process is inhibited at this early stage, it is possible to prevent or counteract subsequent tissue damage or symptoms caused by viral, bacterial, parasitic or fungal attack.

### Description of local anaesthetic substances in relation to the invention

Previous tests have shown that chemical substances belonging to the group of local anaesthetics have, in addition to the local anaesthetic properties, other properties which give rise to a specific inhibition of inflammatory reactions, without thereby adversely affecting the resistance to infection. On the contrary, local anaesthetics can themselves have direct antiviral, antibacterial and antimycotic effects (see, for example, US Patent 587,398 and EP 154,344) which in theory means that the infection itself can be completely cured more quickly. However, a medicament which inhibits the increased vascular permeability and the pathological protein loss occurring in tissue in connection with abnormal temperature conditions, chemical substances and/or radiation is not described.

The present invention or the medicament involves administering pharmacologically active amounts of individual chemical local anaesthetics or mixtures thereof systemically, orally, topically, vaginally or rectally to patients. Pharmaceutically inert vehicles which can be mentioned are, for example, balsam, suspension emulsion, ointment, cream, powder, spray, enema or suppository.

Local anaesthetics are a group of drugs comprising a number of chemical structures. The predominant pharmacological effect of local anaesthetics consists of a reversible blockage of excitation and conduction in peripheral nerves or in the spinal cord, the primary result of which is a reduction in the sensitivity to pain and contact.

The oldest of the substances with a local anaesthetic action is cocaine, whose general structure has been known since 1896 (Willstätter). Since cocaine is unsuitable as a local anaesthetic on account of its dependency-inducing effect, early attempts were made to synthesize local anaesthetics having lower toxicity and fewer side effects. In this work, structures were synthesized which had the same principal structure as the cocaine molecule, i.e. aromatic structure - middle chain - amino group, or even more generally:
lipophilic centre - middle chain - hydrophilic centre.

An aromatic or heterocyclic ring usually functions as the lipophilic centre, but aralkyl or alkyl can also occur.

The middle chain is made up partly by a carbon chain, partly by the groups -COO-, -NH-COO-, -CO-NH-, -NH-CO-, -NH-CO-NH-, -CO- or -O-. Structures with only the carbon chain can also have a local anaesthetic effect.

A tertiary or secondary amino group is generally seen as the hydrophilic centre, if appropriate -OH as in benzyl alcohol. Quaternary nitrogen is not among the structures which are of therapeutic interest as local anaesthetics. It is generally considered that the balance between the hydrophilic and lipophilic properties is of great importance for the local anaesthetic properties. Local anaesthetics are generally classed in the following groups (Pharmacopeia Nordica): esters (for example benzocaine, procaine, tetracaine), amides (for example cinchocaine, lidocaine, prilocaine, mepivacaine, bupivacaine, etidocaine) and ethers (for example chinisocaine, pramocaine).

The local anaesthetic substances primarily concerned in this invention comprise a'group of compounds which have the formulae: 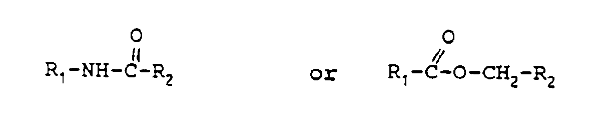
in which R₁ is a substituted benzyl ring with one or more substituents chosen from a group consisting of -Cl, -CH₃, -NH₂, -HNH₉C₄, 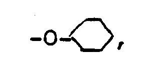
OCH₂CH₂CH₃ or OCH₂CH₂CH₂CH₃, and R₂ is a secondary or tertiary amino group which has been chosen from a group of the following substituents: 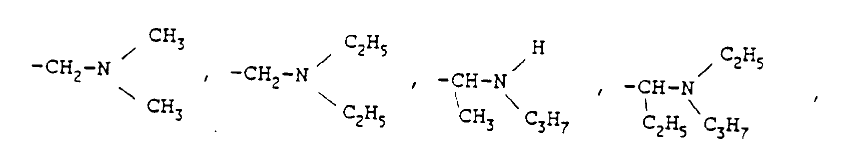 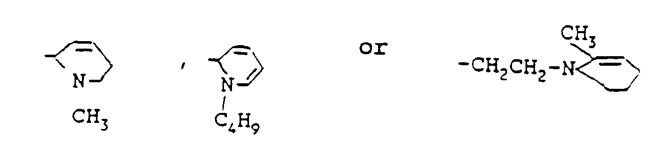
Insofar as these and other chemically related synthetic amides, esters and ethers function in the same way as lidocaine, whose experimental and clinical properties as an inhibitor of pathological vascular permeability and of accompanying plasma loss to tissue are described in detail below, these substances are to be regarded as identical to lidocaine as regards the properties which the invention encompasses and thus come within the scope of this patent application.

### Description of preparation forms:

Lidocaine and its pharmaceutically active salts have been found, according to the invention, to possess pronounced permeability-inhibiting properties in damage to the organism resulting in connection with burn injury, chemical injury and radiation injury. These properties have been observed in experimental animal models and in connection with pathological conditions which have occurred in the event of external injury in humans. The invention lies partly in the knowledge that those properties of lidocaine concerned in this patent application appear within a dosage range considerably below the doses which are used to obtain the local anaesthetic effect. The recommended local anaesthetic doses which have normally been used hitherto depend on the condition or conditions which it is desired to treat, but in the case of systemic administration the dose is generally about 4.3 mg/kg body weight. Thus, in a man weighing 70 kilos, the total recommended daily dose amounts to about 300 mg. In the case of local (topical) administration (usually as an ointment, solution or spray) a 1.5 to 10% preparation is usually given 3 to 4 times a day.

When lidocaine is administered in a pharmaceutically acceptable form, the salt consists of a non-toxic salt, for example a sulphate, nitrate, phosphate, acetate, tartrate, citrate, fumarate, maleate, toluene sulphonate, methane sulphonate or hydrohalide.

Even if it is possible to administer lidocaine or its pharmaceutically acceptable salt forms in substance form, it should however in principle always be given in the form of a pharmaceutical preparation. This pharmaceutical preparation consists of the active substance together with an acceptable vehicle. This vehicle must be "acceptable" in the sense that it will be compatible with the other ingredients included in the preparation and that it will not cause damage or have negative consequences when administered. The preparations concerned in this patent application will be capable of being manufactured by means of known pharmaceutical techniques.

In cases where the pharmaceutical preparation is administered topically in the form of, for example, a solution, spray, ointment, cream or gel, the vehicle can contain one or more of the following substances: petrolatum, lanolin, polyethylene glycols, glycols, beeswax, mineral oil or other suitable vehicles. The preparation can also comprise solvents such as water and alcohol, emulsifiers, stabilizers or other suitable substances. The topical preparations can contain lidocaine or comparable substances specified above in an amount suitably of 1.5 to 10 percent by weight or volume.

Pharmaceutical preparations for parenteral use can comprise sterile solutions or suspensions of the active substance or substances in water or other suitable vehicles.

### Detailed description of the invention:

### Lidocaine in the treatment of pathological vascular permeability and plasma loss to tissue

The invention concerns previously unreported properties of local anaesthetic drugs as defined hereinabove, which properties comprise an inhibition of increased permeability in the blood vessels, which contributes to preventing an increased plasma loss to tissue in connection with external injury or certain pathological conditions. Such tissue damage occurs in connection with abnormal temperature conditions, chemical substances and/or radiation. Since the early damage processes which occur in the tissue as a result of such non-specific action have very great similarities in respect of their mechanism of action, the damage process can be treated as an entity. A large number of different types of agents or processes such as, for example, heat, fire, cold and radiation or microorganisms, for example viruses, bacteria, fungi and parasites, give rise in their very early stages to an increased vascular permeability and accompanying plasma loss to tissue, which constitutes a trigger mechanism for progressive tissue damage.

Insofar as other types of external or internal injury processes or other agents giving rise to the same type of initial tissue damage as burn injuries, chemical injuries or radiation injuries of the skin or internal organs with increased vascular permeability and accompanying plasma loss to tissue are described in detail below, these types of injury are to be considered as identical in respect of the mechanisms of action which the invention encompasses and thus fall within the claims of this patent application.

### 1. Tissue damage caused by abnormal temperature conditions

Tissue injuries occurring when humans or animals are subjected to abnormal temperature conditions are generally covered by the term burn injuries. The most common causes of burn injuries are fire or other abnormally hot objects or liquids. Injuries which are similar to burn injuries occur when tissues are subjected to excessive cold in the event of low environmental temperatures or in the event of accidental direct contact with, for example, parts of the skin. These injuries arising from abnormal temperature conditions result in long periods of treatment in intensive care wards, with a high mortality rate. The therapeutic problem consists in attempting to decrease or stop this substantial loss in order to thereby reduce the considerable fluid and electrolyte imbalance which occurs.

Early administration of local anaesthetics topically in the case of minor burns or intravenously in the case of more extensive burns has a marked effect on the subsequent development of the tissue damage. The studies which have been carried out within the framework of the invention show that local anaesthetics, administered topically on the skin as a cream or ointment, or systemically as an intravenous infusion, can be used in the therapeutic armamentarium in the clinical treatment of burn injuries.

The following experimental systemic studies, clinical tests and case studies document the clinical applicability of local anaesthetics in burn injuries.

### 1.1 Experimental studies on animals

Experimental studies on animals have shown that topical administration, on the skin, of a cream or ointment containing local anaesthetic significantly reduces the loss of Evans blue albumin, which functions as a marker for the initial vascular damage occurring in the burn area. A similar effect is achieved when local anaesthetics are administered as an intravenous infusion.

A detailed description of the test protocol and of the results obtained is given hereinbelow:

### PROTEIN LOSS RESULTING FROM BURNS

### I. Experimental injuries in animals

Tests were carried out on 106 Sprague-Dawley rats. The animals were fasted for 12-15 hours before the experiment. The animals were anaesthetized with 50 mg/kg mebumal i.p., after which anaesthesia was maintained by means of continuous i.v. infusion of chloralose. Tracheal cannulas were inserted, and catheters were introduced into the femoral artery and vein. The body temperature was maintained by using a thermo-regulated hotplate. The skin of the abdomen was shaved carefully, and a burn injury was effected on the abdominal skin using an electrically heated hot-rod (base surface 1 x 1 cm) coupled to an adjustable transformer. A heat-sensitive electrode on the hotplate was coupled to a Grass polygraph for graded temperature control. The plate was immersed in 5 ml of water, and the current was switched on until the water began to boil and the curve levelled out. At temperature level of 100°C was set, after which the current was switched off and the rod was allowed to cool. At a temperature of 55°C the hotplate was brought into contact with the skin of the abdomen until the temperature had fallen to 45°C, after which the contact was broken. This method permits administration of a constant amount of heat energy to the skin independently of the body temperature and blood flow. It was possible to standardize the pressure of the hot-rod on the skin by pressing down a cylindrical handle which compresses a spring on the hot-rod. The room temperature varied between 21 and 23°C.

In the first experimental group (n=15) the burned skin was treated with lidocaine/prilocaine cream (25 mg of each local anaesthetic in 1 g) for 15 minutes following the burn injury. The cream was allowed to act for 1½ hours. In the control group, the animals were subjected to the same type of burn injury, while the skin was treated with a placebo cream with the same pH and composition, but without local anaesthetic. In a third group, the animals were not subjected to any burn injury and were used here as a reference for normal skin.

In a separate series of experiments, the animals were given an intravenous injection of lidocaine (2 mg/kg) followed by a continuous intravenous infusion of lidocaine at an infusion rate of 5 (n=10), 10 (n=12), 20 (n=10) or 30 (n=10) µg kg⁻¹ min⁻¹ for 1½ hours. The infusion was started 15 minutes following the burn injury. One control group with burns and one control group without burns received corresponding injections and infusions of saline. All volumes were administered at a rate of 0.6 ± 0.03 ml/hour.

At the end of all the tests, Evans blue (EB) was injected intravenously (20 mg/kg) dissolved in 1 ml of NaCl. EB binds to albumin in plasma and functions as a marker for this in cases of extravascular loss. The animals were sacrificed 30 minutes later using saturated KCl. The skin area with the burn injury (1x1 cm) was cut out (full skin thickness), dried on filter paper and weighed. Nach tissue sample was placed in 4 ml of formamide and incubated for 24 hours in a water bath at 50°C. The EB-albumin loss to tissue was measured at 612 nm. Three measurements were carried out on each tissue sample. Calculations were carried out by comparison with an external standard in formamide. All the tissue analyses were carried out by a laboratory assistant not informed of the details of the test protocol.

In a separate series of animals (in addition to the 106 animals described above) histological examination was carried out to determine the EB-albumin loss in burned tissue by means of fluorescence microscopy. Thirty minutes after injection of EB, the burned skin area was cut out and fixed in 4% formaldehyde for 24 h. After this procedure which was carried out on 5 animals from each group, corresponding to the groups described above, the samples were frozen in liquid nitrogen and cut into 10µm thick sections which were then heat-dried at 30°C for 1 minute. The sections were examined under a fluorescence microscope (Microphot-FX, Nikon) using the epi-illumination technique (Ploek Pam System) consisting of a UV lamp, excitation filter (450-490 nm) and mirror and suppression filter.

### RESULTS:

Burned skin exhibited significantly greater loss of albumin compared to unburned skin (p<0.001). Topical administration of lidocaine/prilocaine cream on burned skin produced a significant and very pronounced inhibition of plasma albumin loss (p<0.001). This inhibition was of such an extent that there was no significant difference in plasma albumin loss between burned skin treated with topical local anaesthetics and unburned skin.

In the group in which lidocaine was administered as a continuous intravenous infusion, the albumin loss was significantly reduced at 10 and 20 µg kg⁻¹ min⁻¹ (p<0.01). At a lidocaine infusion of 10 µg kg⁻¹ min⁻¹, there was no significant difference in plasma albumin loss compared to unburned skin. No cardiovascular effects were detected during the lidocaine infusions, i.e. the effect on burn injuries was observed upon therapeutic infusion of lidocaine.

Fluorescence microscopy showed red fluorescence where EB-albumin had leaked out into free tissue. In unburned skin the fluorescence was found in the lumen of large blood vessels. In the case of burned skin, a considerable loss could be seen in all layers of the skin. Topical and systemic administration of a local anaesthetic significantly reduced this loss.

### 1.2. Clinical case reports

### CONTINUOUS LIDOCAINE INFUSION IN BURN INJURIES

### Case report: Patient No. 1:

A 44 year old male, previously healthy. No medicines and no allergies. Works for a company which services and repairs steam furnaces. On the day of the accident the patient was lying under a so-called low-pressure furnace, which he wrongly thought empty. When he opened the valve, he was hit by water at 90°C over the front of the trunk, right arm and thigh. Upon admission to hospital, he was assessed as having second degree burns covering about 10% of the body surface. There was extensive blister formation over the burned surfaces. In pain upon admission, writhing on the stretcher. 97 points on the pain scale (VAP - visual analog pain scale: 0= no pain, 100= unbearable pain). Received intramuscular injection of 75 mg pethidine, with little alleviating effect on the pain after 1 hour. Given the extensive blister formation and the pain, the patient was put on lidocaine. He received a 100 mg i.v. injection of lidocaine, followed by continuous intravenous infusion of lidocaine at a rate of 3 mg/min for a total of 3 days. The plasma loss from the burn area was drastically reduced as early as the second day, and it was possible to reduce the number of dressings from 11 on the first day to 3 on the second and third days. The loss usually increases during the first days following the burn injury, and then slowly decreases in the course of 1-2 weeks. In addition, the patient experienced almost complete freedom from pain during the period of the lidocaine infusion (mean of accumulated pain score = 8!). No morphine product was required during the infusion. When infusion was stopped, the plasma loss remained inhibited, but the pain returned with high intensity.

### Case report: Patient No. 2:

A 45 year old male, who had previously been essentially healthy, was admitted with second degree burns over the front of the trunk and on the arms and hands (10% of total body surface). The patient presented severe blister formation in the burned areas. Moreover, upon admission, the patient was in great pain. He was given intramuscular pethidine, without a sufficient analgesic effect. He received intravenous injection of 100 mg lidocaine, followed by a continuous intravenous infusion of lidocaine at a rate of 3 mg/min over the course of 3½ days (therapeutic dose for heart arrhythmia). As in the preceding case, the patient responded with a rapid decrease in plasma loss from the burn blisters and with almost complete freedom from pain. The draining effect on the burn blisters remained after infusion was stopped, but the pain returned, albeit to a lesser extent than in the preceding case.

### 1.3. Clinical controlled studies on burn patients

### BURNS RESULTING FROM ELECTROVERSION

In electroversion, a defibrillator is used to apply one or more current pulses to the thoracic cage. In addition to the, effect on the heart rhythm, these pulses also result in a local first-degree burning of the skin which causes the patient pain and skin irritation. The degree of the burn depends on the amount of energy supplied, which varies between 100 and 300 Ws. With the aid of a new computerized technique (Computer Assisted Photograph Analysis), the degree of increase in blood flow in the burn area and the subsequent oedema formation are assessed. The method has a high resolution. In an assessment of 10 patients, 5 patients received lidocaine/prilocaine cream on the skin where the positive electrode plate was placed about 1 hour before electroversion, while 5 patients received a placebo cream in a corresponding manner. The results show an almost complete inhibition of redness and oedema formation under the electrode plate compared to the placebo group. In addition, the lidocaine/prilocaine group experienced complete freedom from pain during the 4-hour long observation period.

### 1.3.2.

### BURNS IN TEST VOLUNTEERS

Using the same technique described above for experimental burns in animals, we produced a first-degree burn on the inside of the forearm of test volunteers by means of a hot-rod (burn surface 1x1 cm). A group of 3 individuals received 5% lidocaine/prilocaine cream, applied to the skin 5 min after burning, and placebo cream was applied to the same individuals at a later occasion. The burn was photographed by the CAPA technique and was analyzed in respect of redness and oedema formation. The results show that application of a local anaesthetic after burning also produces a pronounced inhibition of redness and oedema formation.

### 2. Tissue damage caused by chemical substances

Tissue damage can be caused by a number of different chemical substances which by their chemical nature are acids or bases and which, upon contact with the skin or internal organs, can trigger an increased vascular permeability and accompanying plasma loss to tissue. Other chemical substances can cause a release of biochemical peptides in the tissue, for example bradykinin, histamine, serotonin, prostaglandins or leukotrienes and can in this way initiate changes in permeability and leakage.

Chemical tissue damage can therefore occur when tissue-irritating chemical substances come into contact with the skin or internal organs, particularly in the abdominal cavity or thoracic cage. The chemical substances which initiate the damage can come from the body itself, such as, for example, hydrochloric acid in the stomach, bicarbonate in the small intestine, or bile salts in the gallbladder. It can of course also be external chemical substances which come into contact with skin or internal organs in connection with accidental or non-accidental injury. A common form of non-accidental or iatrogenic injury consists of surgical operations in which small amounts of exogenous substances come into contact with the operation wound, peritoneum, pleura etc. and can thus trigger an increased vascular permeability and accompanying plasma loss to tissue. This initial process can then be the cause of inflammation of the peritoneum (chemical peritonitis) or serious conditions involving complete blockage of the intestine (occlusive ileus) with excessive loss from circulation to tissue. In some cases, chemical irritation in the urogenital organs (interstitial cystitis) can give rise to basically the same type of tissue damage.

### 2.1. Experimental studies on animals

Experimental studies of chemical tissue damage can be carried out by means of administration of irritating chemical compounds to the skin, abdominal cavity or thoracic cavity. The effects of lidocaine according to the invention were observed in an animal model which simulates chemical peritonitis in humans.

### 2.1.1. Chemical peritonitis

Chemical peritonitis occurs clinically, for example in cases of acute pancreatitis or perforated gastric ulcer, resulting in loss of plasma from the circulation to the free abdominal cavity. This in turn leads to a disturbed fluid and electrolyte balance in already extremely sick patients with poor prognosis. Experimental studies carried out within the framework of the invention have shown that topical administration of local anaesthetics to the peritoneum before induction of chemical peritonitis almost completely prevents increased vascular permeability and the accompanying plasma loss to tissue. These studies have also shown that the administration of local anaesthetic agents after peritonitis has been triggered significantly inhibits the loss of Evans blue albumin as a measure of plasma loss to tissue.

A detailed description of the test protocol and of the results obtained in these tests is given hereinbelow:

### ALBUMIN LOSS IN PERITONITIS

### I. Experimental tests on animals

In all the tests the abdominal wall was opened with a midline incision down to the abdominal cavity. The colon and its peritoneum-covered surface were exposed. This study assessed the effect of lidocaine and bupivacaine on peritonitis triggered with hydrochloric acid (HCl).

### Experimental model 1:

A piece of gauze (1.5 x 1.5 cm) containing 1% lidocaine hydrochloride (pH 6.9;n=8) or 0.5% bupivacaine hydrochloride (pH 7.1; n=8) was placed on the peritoneum for 5 minutes. A control group was treated in a corresponding manner for 5 minutes with gauze containing sodium chloride. The same area of the peritoneum was thereafter subjected to hydrochloric acid by means of a piece of gauze (1x1 cm) containing 0.5 ml of 0.1 M HCl being placed on the peritoneum.

### Experimental model 2:

Here peritonitis was triggered first by placing gauze containing hydrochloric acid, as described above, on the peritoneum for 5 minutes. The peritonitis was allowed to develop for 15 minutes, after which 3 groups were treated for 5 minutes with gauze containing 1% lidocaine hydrochloride (pH 6.9;n=8), 0.5% bupivacaine hydrochloride (pH 7.0;n=8) or sodium chloride (pH 7.0;n=7).

Fifteen minutes after the procedure in models 1 and 2, Evans blue (EB) was injected intravenously (20 mg/kg) dissolved in 1 ml of NaCl. EB binds to albumin in plasma and functions as a marker for this in cases of extravascular loss. Five minutes later the superior mesenteric artery was catheterized and the superior mesenteric vein was cut away, after which the vascular bed of the colon was flushed with 20 ml of saline (37°C) in order to eliminate all EB albumin which had not leaked out to the free tissue. The animals were sacrificed with saturated KCl. The surface of the intestine exposed to hydrochloric acid was cut out, dried on filter paper and weighed. Each tissue sample was placed in 4 ml of formamide and incubated for 24 hours in a water bath at 50°C. The loss of EB-albumin to tissue was measured at 612 nm. Three measurements were carried out on each tissue sample. Calculations were carried out by comparison with an external standard in formamide. All the tissue analyses were carried out by a laboratory assistant not informed of the details of the test protocol.

### Experimental model 3:

A histological examination was carried out on a separate series of animals in order to determine the EB-albumin loss in burned tissue by means of fluorescence microscopy. Five minutes after administration of EB, the experimental area was cut out and fixed in 4% formaldehyde for 24 h. After this procedure, the samples were frozen in liquid nitrogen and cut into 10-µm thick sections which were then heat-dried at 30°C for 1 minute. The sections were examined under a fluorescence microscope (Microphot-FX, Nikon) using the epi-illumination technique (Ploek Pam System) consisting of a UV lamp, excitation filter (450-490 nm) and a mirror and suppression filter. Photographs were taken using ASA 160 film (Kodak ektachrome 135-36).

### Experimental model 4:

In a group of 5 animals, the peritoneum was treated for 5 min with 0.5% ³H-bupivacaine (470 mCi/mmol). The experimental area was then cut out and immediately immersed in liquid nitrogen. The preparation was then fixed and was cut into 20-µm thick sections in a cryostat, transferred to a glass plate and heated to 70°C for 30 s. An X-ray film (Kodak DF-57) was placed over the sections at room temperature and was exposed for 4-8 days. The position of the ³H-bupivacaine was detected on the X-ray film and was compared to the corresponding histological sections.

### RESULTS:

Hydrochloric acid on the peritoneum (peritonitis) triggered a pronounced loss of albumin. When 1% lidocaine was administered to the peritoneum before administration of HCl, there was a significant and pronounced inhibition of albumin loss (p<0.01). No significant difference was found between lidocaine-treated peritoneum subjected to HCl and peritoneum not treated with hydrochloric acid. Similar results were obtained when 0.5% bupivacaine was administered before the hydrochloric acid.

When lidocaine and bupivacaine were administered 5 min after the peritoneum had been subjected to the hydrochloric acid, here too there was a pronounced inhibition of plasma loss compared with administration of saline.

Fluorescence histology showed a considerable extra-vascularization of albumin in the peritoneum and underlying tissue. Treatment with lidocaine and bupivacaine both before and after exposure to hydrochloric acid greatly inhibited the loss, which it was possible to observe with considerably weaker fluorescence in free tissue compared with the control.

Autoradiography of the peritoneum and colon showed that isotope-labelled bupivacaine penetrates down to the muscularis interna but does not penetrate further into the submucosa, i.e. covers well the area from which the albumin loss takes place.

Cortisone-containing solutions have hitherto been used in clinical contexts in order to wash the peritoneum, with the risk of serious systemic side effects upon repeated treatment. No such side effects have been observed for local anaesthetic drugs within the dose range in question, for which reason this method of treatment,' which has also been shown to be more effective than cortisone, is to be preferred in clinical use.

### 2.1.2 Occlusive ileus

Blockage of the small intestine as a result of fusion in the abdomen, for example after previous abdominal surgery, so-called occlusive ileus, leads to a life-threatening condition with serious fluid and electrolyte losses to the intestine and dehydration of the patient. The mortality associated with this condition is still very high. No previously known therapy has been found to be able to reduce the great fluid losses before and after surgery. Experimental studies on animals have shown that local anaesthetics administered topically on the occluded section of the intestine or intravenously reduce the inflammation in the occluded intestine and thereby reduce the serious fluid losses compared to saline placebo.

A detailed description of the test protocol and of the results obtained in these tests is given hereinbelow:

### ALBUMIN LOSS IN OCCLUSIVE ILEUS

The tests were carried out on Sprague Dawley rats which had been dehydrated for 12 hours. The animals were anaesthetized with brietal, after which the abdomen was opened and a ligature was established round the jejunum 10 cm distal of Treitz' ligament. The abdomen was then closed using synthetic sutures, and the animals were allowed to recover. After 18 hours the animals were anaesthetized once more with i.p. mebumal, and catheters were inserted into the femoral artery and into the femoral vein. The animals were tracheotomized. The abdomen was opened again, and the occluded segment of the small intestine was studied.

Evans blue (EB) (20 mg/kg) dissolved in 1 ml of NaCl was injected intravenously. EB binds to albumin in plasma and functions as a marker for this in cases of extravascular loss. Five minutes later, the superior mesenteric artery was catheterized and the superior mesenteric vein was cut away, after which the vascular bed in the small intestine was flushed with 20 ml of saline (37°C) in order to eliminate all EB-albumin which had not leaked out to the free tissue. The animals were sacrificed with saturated KCl. A piece of the occluded small intestine was cut out, dried on filter paper and weighed. Each tissue sample was placed in 4 ml of formamide and incubated for 24 hours in a water bath at 50°C. The loss of EB-albumin to tissue was measured at 612 nm. Three measurements were carried out on each tissue sample. Calculations were carried out by comparison with an external standard in formamide. All tissue analyses were carried out by a laboratory assistant not informed of the details of the test protocol.

Histological examination of the same animals was carried out in order to determine the EB-albumin loss in burned tissue by means of fluorescence microscopy. Five minutes after administration of EB, part of the small intestine was cut out and fixed in 4% formaldehyde for 24 hours. After this procedure, the samples were frozen in liquid nitrogen and cut into 10-µm thick sections which were then heat-dried at 30°C for 1 minute. The sections were examined under a fluorescence microscope (Microphot-FX, Nikon) using the epi-illimination technique (Ploek Pam System) consisting of a UV lamp, excitation filter (450-490 nm) and a mirror and suppression filter.

One group was treated with 10% lidocaine aerosol on the section of the small intestine proximal of the ligature made in the first operation. A control group was treated with placebo aerosol, which was identical but did not contain lidocaine.

### RESULTS

The albumin loss in the control group treated with placebo aerosol was very pronounced. This was demonstrated quantitatively by means of a spectrophotometer and qualitatively by means of fluorescence microscopy. In the group which was treated with lidocaine, there was a pronounced and significant inhibition of albumin loss to free intestinal tissue.

No cardiovascular effects were observed in the animals. The results illustrate the inhibitory power by demonstrating the same degree of albumin loss in normal, non-occluded small intestine as in an occluded small intestine treated with local anaesthetic drugs.

Thus, continuous systemic administration of a suitable preparation of lidocaine or other local anaesthetic drugs in accordance with the above can be started as soon as the diagnosis is established by X-ray and can be continued during the whole period of treatment, given the low rate of side effects with these drugs. Local anaesthetics can also be administered topically in a suitable preparation form, for prophylactic or therapeutic purposes, in patients undergoing surgical intervention in the abdominal or thoracic cavity.

### 2.2. Interstitial cystitis

Interstitial cystitis is a severe irritation of the urinary bladder, involving swelling of the mucous membrane and muscle wall of the urinary bladder, which in turn causes the patient severe pain and abnormally small bladder volumes. No effective symptomatic treatment is available at present, and for this reason many patients finally undergo a Bricker operation in which the bladder is removed and the intestine is made to act as a bladder. The effect of lidocaine on the fluid loss and the swelling in the wall of the urinary bladder of patients with interstitial cystitis can be illustrated here on the basis of a case report:
(B. Asklin and J. Cassuto, "Intravesical lidocaine in severe interstitial cystitis", in publication) A woman with interstitial cystitis and severe pain in the urinary tract was treated experimentally with lidocaine. Before treatment she had a total bladder volume of 50 ml, for which reason she was forced to empty the bladder into diapers during the night (cannot have KAD on account of pain). Treatment was started with intravesical installation of local anaesthetic agents daily into the bladder via a cystoscope under local anaesthesia. After 5 days it was possible to reduce the treatment to every third day. Three weeks later the woman's bladder volume had increased to 250 ml and cystoscopic examination showed a very pronounced reduction in the inflammation of the mucous memhrane of the patient's bladder. The woman remained virtually free of symptoms and was able to continue treatment at home.

## Claims

1. Use of a chemical local anaesthetic agent selected from the local anaesthetic compound groups:
- esters, for example BENZOCAINE, PROCAINE and TETRACAINE,
- amides, for example CINCHOCAINE, LIDOCAINE, PRILOCAINE, MEPIVACAINE, BUPIVACAINE and ETIDOCAINE,
- ethers, for example CHINISOCAINE and PRAMOCAINE,
for the production of a medicament for prophylactic or therapeutic inhibition of increased vascular permeability in the blood vessels, which contributes to preventing increased plasma loss to tissue in the case of burn injuries, cold injuries or chemical injuries of the skin or internal organs, in particular in the abdominal cavity or thoracic cage.

2. Use of a chemical local anaesthetic agent according to claim 1, **characterized** in that it is present in base form or as a pharmaceutically acceptable salt.

3. Use of a chemical local anaesthetic agent according to any one of claims 1 - 2, **characterized** in that it contains one or more inert vehicles for systemic, i.e. infiltration, intravascular (intravenous or intraarterial), intraarticular, epidural and/or intrathecal administration to patients.

4. Use of a chemical local anaesthetic agent according to any one of claims 1 - 3, **characterized** in that it contains one or more inert vehicles for topical administration to patients.

5. Use of a chemical local anaesthetic agent according to claim 4, **characterized** in that the pharmaceutically inert vehicle comprises an alcohol, balsam, suspension, emulsion, ointment, cream, powder or spray.

6. Use of a chemical local anaesthetic agent according to any one of claims 1 - 2, characterized in that it contains one or more inert vehicles for oral administration to patients.

7. Use of a chemical local anaesthetic agent according to any one of claims 1 - 2, **characterized** in that it is formed into a preparation for rectal, vaginal or vesical administration.

8. Use of a chemical local anaesthetic agent according to any one of claims 3 - 6, **characterized** in that it is formed into or contained in a medium with sustained dissolution or administration, by means of which the medicament is able to act for a prolonged period of time after administration.

9. Use of a chemical local anaesthetic agent according to claim 4, **characterized** in that it is formed into a shampooing agent.

## Patentansprüche

1. Verwendung eines chemischen örtlich narkotischen Mittels, welches aus den örtlich narkotischen zusammengesetzten Gruppen ausgewählt ist:
- Ester, z.B. Benzocaine, Procaine und Tetracaine,
- Amide, z.B. Cinchocaine, Lidocaine, Prilocaine, Mepivacaine, Bupivacaine und Etidocaine,
- Ester, z.B. Chinisocaine und Pramocaine,
zur Herstellung eines Medikaments für die prophylaktische oder therapeutische Hemmung von anwachsender Gefäßdurchlässigkeit in den Blutgefäßen, welches zur Vermeidung von anwachsendem Plasmaverlust in das Zellgewebe beiträgt in den Fällen von Brandverletzungen, Kälteverletzungen oder chemischen Verletzungen auf der Haut oder von inneren Organen, insbesondere in der Bauchhöhle oder dem Brustkorb.

2. Verwendung eines chemischen örtlich narkotischen Mittels gemäß Anspruch 1, dadurch gekennzeichnet, daß es in Basenform oder als pharmazeutisch akzeptables Salz vorliegt.

3. Verwendung eines chemischen örtlich narkotischen Mittels nach einem der Ansprüche 1 bis 2, dadurch gekennzeichnet, daß es ein oder mehrere inerte Vehikel zur systematischen Verabreichung an den Patienten, z.B. durch Infiltration, in die Gefäße (intravenös oder intraarteriell), in die Gelenke, in die Oberhaut und/oder intrathecal enthält.

4. Verwendung eines chemischen örtlich narkotischen Mittels nach einem der Ansprüche 1 bis 3, dadurch gekennzeichnet, daß es ein oder mehrere inerte Vehikel für die örtliche Verabreichung an den Patienten enthält.

5. Verwendung eines chemischen örtlich narkotischen Mittels nach Anspruch 4, dadurch gekennzeichnet, daß das pharmazeutische inerte Vehikel einen Alkohol, einen Balsam, eine Suspension, eine Emulsion, eine Salbe, eine Creme, ein Puder oder ein Spray umfaßt.

6. Verwendung eines chemischen örtlich narkotischen Mittels nach einem der Ansprüche 1 bis 2, dadurch gekennzeichnet, daß es ein oder mehrere inerte Vehikel für die orale Verabreichung an den Patienten enthält.

7. Verwendung eines chemischen örtlich narkotischen Mittels nach einem der Ansprüche 1 bis 2, dadurch gekennzeichnet, daß es als Präparat für die rektale, vaginale oder Blasenverabreichtung ausgebildet ist.

8. Verwendung eines chemischen örtlich narkotischen Mittels nach einem der Ansprüche 3 bis 6, dadurch gekennzeichnet, daß es in einem Medium mit andauernder Auflösung oder Verabreichung ausgebildet oder darin enthalten ist, [und zwar] durch Mittel, mit welchen das Medikament fähig ist, für einen verlängerten Zeitraum nach der Verabreichung zu wirken.

9. Verwendung eines chemischen örtlich narkotischen Mittels nach Anspruch 4, dadurch gekennzeichnet, daß es in einem Schampuniermittel ausgebildet ist.

## Revendications

1. Emploi d'un anesthésique local chimique, sélectionné parmi les groupes de composés anesthésiques locaux:
- esters, par exemple BENZOCAINE, PROCAINE et TETRACAINE,
- amides, par exemple CINCHOCAINE, LIDOCAINE, PRILOCAINE, MEPIVACAINE, BUPIVACAINE et ETIDOCAINE,
- éthers, par exemple QUINOSOCAINE et PRAMOCAINE,
pour la production d'un médicament destiné à l'inhibition prophylactique ou thérapeutique de l'hyperméabilité vasculaire des vaisseaux sanguins, lequel contribue à prévenir les déperditions excessives de plasma dans les tissus dans le cas de lésions par brûlures, de lésions dues au froid ou de lésions chimiques de la peau ou des viscères, notamment dans la cavité abdominale et/ou la cage thoracique.

2. Emploi d'un anesthésique local chimique selon la revendication 1, caractérisé en ce qu'il est présent sous la forme d'une base ou d'un sel pharmaceutiquement acceptable.

3. Emploi d'un anesthésique local chimique selon l'une quelconque des revendications 1 à 2, caractérisé en ce qu'il contient un ou plusieurs véhicules inertes destinés à l'administration systémique, c'est-à-dire infiltration, intravasculaire (intraveineuse ou intraartérielle), intraarticulaire, épidurale et/ou intrathécale chez des malades.

4. Emploi d'un anesthésique local chimique selon l'une quelconque des revendications 1 à 3, caractérisé en ce qu'il contient un ou plusieurs véhicules inertes destinés à l'administration topique chez les malades.

5. Emploi d'un anesthésique local chimique selon la revendication 4, caractérisé en ce que le véhicule pharmaceutiquement inerte comprend un alcool, baume, suspension, émulsion, pommade, crème, poudre ou spray.

6. Emploi d'un anesthésique local chimique selon l'une quelconque des revendications 1 à 2, caractérisé en ce qu'il contient un ou plusieurs véhicules inertes destinés à l'administration orale chez des malades.

7. Emploi d'un anesthésique local chimique selon l'une quelconque des revendications 1 à 2, caractérisé en ce qu'il se présente sous la forme d'une préparation destinée à l'administration rectale, vaginale ou vésicale.

8. Emploi d'un anesthésique local chimique selon l'une quelconque des revendications 3 à 6, caractérisé en ce qu'il se présente sous la forme d'un milieu ou est contenu dans un milieu à dissolution ou administration prolongée, permettant au médicament d'agir pendant une durée prolongée après l'administration.

9. Emploi d'un anesthésique local chimique selon la revendication 4, caractérisé en ce qu'il seprésente sous la forme d'un shampoing.
